# EUROPEAN PATENT SPECIFICATION

(11) **EP 0 720 442 B1**
(45) Date of publication and mention of the grant of the patent: **07.01.2004**
(21) Application number: 94929247.8
(22) Date of filing: 20.09.1994
(51) Int. Cl.: A61B 5/00, A61B 10/00

(54) **MULTIPLE BIOPSY SAMPLING CORING DEVICE**
VORRICHTUNG ZUR MEHRFACH-ENTNAHME VON BIOPSIE-PROBEN
DISPOSITIF A BIOPSIE PERMETTANT DE PRATIQUER PLUSIEURS PRELEVEMENTS SELON LA TECHNIQUE DU CAROTTAGE

(30) Priority: 20.09.1993 US 124272; 30.09.1993 US 129653; 29.10.1993 US 146447
(43) Date of publication of application: 10.07.1996
(60) Divisional application: 02102018.5
(73) Proprietor: Boston Scientific Corporation, Natick, Massachusetts 01760 (US)
(72) Inventor: ROBINSON, Donald E., Hopkinton, MA 01748 (US); BANIK, Michael S., Cincinnati, OH 45241 (US)
(74) Representative: Brunner, Michael John
(86) International application number: PCT/US1994/010565
(87) International publication number: WO 1995/008291

(56) References cited:
- EP-A- 0 065 054
- EP-A- 0 207 829
- WO-A-92/10974
- FR-A- 2 479 680
- FR-A- 2 610 508
- FR-A- 2 625 429
- US-A- 2 541 542
- US-A- 4 461 305
- US-A- 4 651 752
- US-A- 4 785 826
- US-A- 5 111 828
- US-A- 5 133 360
- US-A- 5 148 813
- US-A- 5 195 533
- US-A- 5 197 484

## Description

This invention relates to taking samples of tissue from the body for biopsy analysis.

Tissue samples can be examined in a laboratory to determine the presence of a pathological disorder (e.g. malignancy). Often, the samples must be obtained from deep within the body using a medical sampling instrument. It is usually best to obtain several samples around the location where the disorder is suspected so that the presence and progress of disease, if any, can be accurately determined. The samples must be catalogued according to the location from which each sample is taken and the integrity of the samples must be maintained for the subsequent laboratory analysis.

US 4,785,826 discloses an instrument used to gather tissue. The device retains a gathered tissue specimen by closing the end of a hollow member while the member is in the gathering position.

According to the present invention there is provided a device for collecting a sample of tissue from a tissue surface, comprising:
a device body having a distal end defining a forward-facing tissue receiving opening through which tissue passes when said opening is near said tissue surface, and
a storage space proximal of and adjacent said tissue receiving opening for storage of multiple tissue samples; characterised in that
a set of movable, jaw-like cutting members is disposed over said device body, actuatable to be opened and closed across said tissue receiving opening when tissue from said surface extends through said tissue receiving opening for severing said tissue from said surface, whereby by repeatedly passing tissue through said tissue receiving opening and actuating said cutting members, multiple tissue samples can be stored within said storage space.

Preferably, the cutting members are coaxially disposed and axially positionable with respect to said tissue receiving opening such that the device is positionable between a first configuration in which the cutting members are proximal of said opening for receiving tissue through said opening and a second configuration in which the cutting members are near said opening where said cutting members can be closed to sever tissue and take said sample.

The cutting members may be biased closed, and may be opened by disposing said device in the first configuration, where said cutting members bear on outer surfaces of the body which oppose the force of said bias, and said cutting members may be closed by disposing said device in the second configuration where said cutting members are free from said body and close in response to said bias force.

The tissue receiving opening may be defined about its periphery by a tissue-cutting edge, said tissue passing through said opening by urging said edge distally into said tissue surface.

Preferably the device further comprises an axially movable retractor for engaging said tissue and drawing it proximally.

The retractor may be extendable distally beyond said opening for engaging said tissue surface and retractable proximally to draw tissue through said tissue receiving opening.

The retractor may be a spear-form element adapted to pierce tissue and has a retaining barb on its distal end.

The invention has many advantages. For example, sampling into tissue to a desired depth can be achieved by controlling the amount of sample that enters the forward-facing coring opening of the device, by, for example, controlling how deeply the device is advanced into a tissue wall or how much tissue is drawn through the opening using a retractor. Thick samples can be taken in a single sampling action; for example, samples beyond the mucosal layer (submucosal sampling) can be taken from a site in a single sampling action. Careful control of sampling depth also permits samples to be taken from very thin tissue walls without puncturing the walls. Multiple samples can be taken, stored in the device, and maintained in a hydrated state without removing the device from the body.

An example of the present invention will now be described with reference to the drawings, in which:
Fig. 1 is a perspective view of an embodiment of the invention being delivered into the body through an endoscope;
Figs. 2-2e illustrate the structure and use of an embodiment of the invention;
Fig. 3 illustrates the structure and use of another embodiment of the invention.

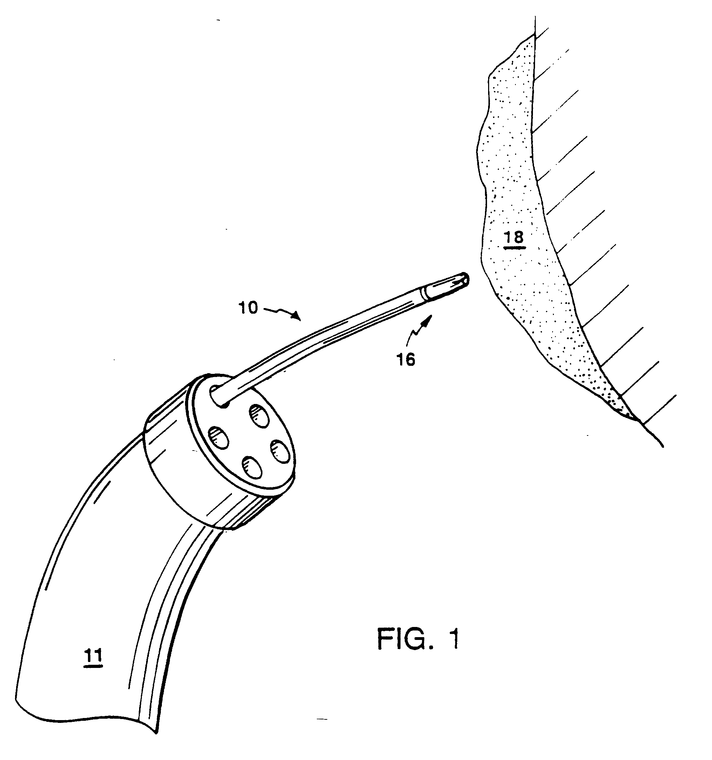

Referring to Fig. 1, the device 10 for multiple biopsy sampling may be delivered into the body through the channel of an endoscope device 11 (e.g., gastroscope, sigmoidoscope, or colonoscope). The endoscope device typically has a length of about 100-250 cm and a channel diameter of 2.0 - 3.8 mm, typically about 2.8 mm. A distal sampling portion 16 is extended from the endoscope for cutting and storing a sample of tissue from a body surface 18 of a patient (e.g. from a surface in the gastrointestinal tract or bronchial tract). The device has a diameter of preferably around 1.8 - 2.4 mm, typically about 2.3 mm or less and is of sufficient flexibility so it passes easily though the channel when the endoscope follows a tortuous body passageway. The endoscope includes other lumens for water, air, suction, and viewing. Devices according to the invention can be adapted to be introduced to sites (e.g., urinary tract, reproductive organs, cardiac tissue, or the like) deep within the body by other means. For example, a device can be configured with a lumen so that it can be advanced over a guidewire, e.g., in vascular applications. The device may be passed through an introducer or guiding catheter in, e.g., cardiac applications. The sampling and storage arrangements may be useful in open surgery applications, in breast biopsy in which the device is pressed directly into tissue, laproscopic biopsy in which the cutting element is positioned through a tubular instrument extending through the skin, and percutaneous needle biopsy in which the device is directed through a hole in the skin to sample an internal organ, e.g., the liver.

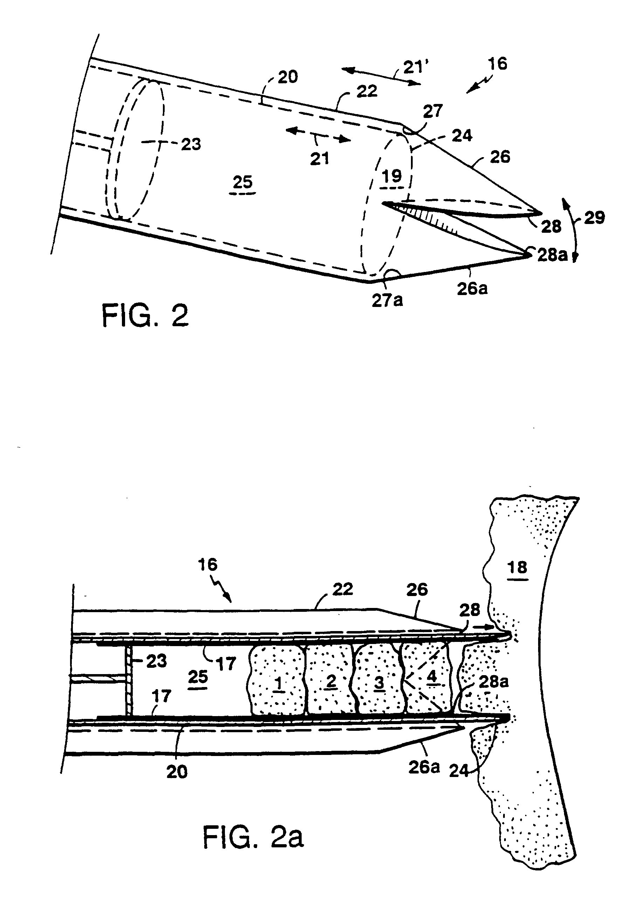

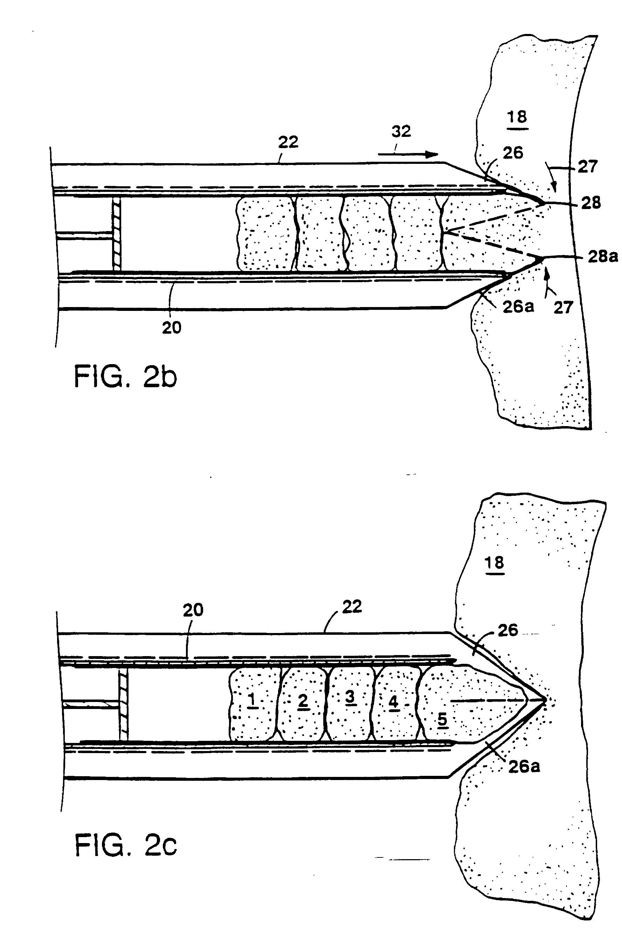

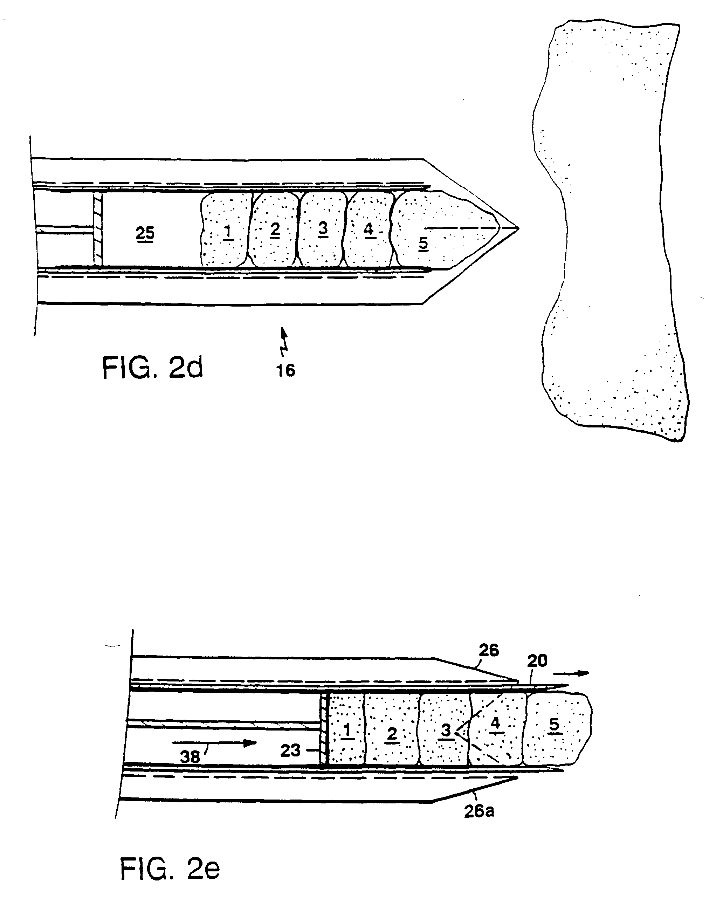

Referring to Figs. 2-2e, in an. embodiment, sampling portion 16 includes an inner tubular sample holding and coring member 20 and an outer cutting member 22. The inner tubular member 20 defines in its proximal portions an inner space 25 for storage of multiple, successively taken biopsy samples. As shown particularly in Figs. 2a et seq., the samples are stored adjacent one another in the order in which they are taken. A sample stop 23 defines the most proximal end of the space 25. The stop 23 can be moved axially distally to retrieve the multiple samples after the device is removed from the body, as will be discussed in more detail below. The distal end of the inner tubular member defines a forward- facing, tissue-receiving opening 19 and is sharpened to a cutting edge 24.

The outer cutting member 22 includes near its distal end a pair of moveable jaw-like cutting elements 26, 26a. The cutting elements are formed of a material having substantial elasticity, for example, a shape memory alloy or stainless steel, and worked such that the cutting elements are biased toward the closed position. The cutting member 22 and the inner tubular member 20 are axially movable with respect to one another (arrows 21, 21'). In the configuration shown in Fig. 2, with the inner tubular member extended distally somewhat with respect to the cutting member 22, the distal end of the cutting member bears on the inner surfaces 27, 27a of the elements 26, 26a, moving them radially into an open position (arrow 29). The outer edges 28, 28a of the cutting elements are sharpened for cutting tissue, as will be further described below.

In embodiments, other cutting element arrangements are possible; for example, elements that rotate about a pivot point and are biased by a spring could be provided. Arrangements with more than two cutting elements may also be used. One of either the cutting member or tubular member may be moveable and the other stationary. The components that experience sliding motion may include a lubricant. For example, the interior wall of the inner tubular member may include a low friction coating 17, e.g., of teflon, silicone, or a hydrogel, so that samples within the tube and storage space slide easily. The outer surface of the inner tubular member and/or the inner surface of the outer tubular member may also include a lubricant to ease sliding motion. (Other sliding components in other embodiments, shown below, e.g., control wires and cutting loops may also include a lubricant.)

Referring particularly to Fig. 2a, (cross-sectional view), in use, the inner tubular member is extended distally to open the cutting elements and extend beyond them so the edge 24 cuts into a surface 18 of tissue to a depth that approximates the desired depth to which a sample is to be taken. The tubular member is rotated slightly about its axis as it is urged forward to create a shearing action that aids cutting.

Referring particularly to Fig. 2b, the sample is severed from the tissue surface 18 by extending the outer cutting member 22 distally (arrow 32). When the cutting member 22 has extended sufficiently beyond the distal edge of the inner tubular member 20, the cutting elements 26, 26a begin to close (arrows 27) and the cutting edges 28, 28a sever the tissue sample from the body surface 18. The cutting member may be rotated slightly about its axis as it is extended forward to create a shearing action to facilitate cutting. As tissue enters the inner tubular member it pushes the previously-taken samples, samples 1- 4, proximally in the space 25.

Referring particularly to Fig. 2c, after the cutting member 22 has been extended distally such that the cutting elements 26, 26a are completely closed, the new sample, sample 5, is cut completely free of the tissue surface 18.

Referring particularly to Fig. 2d, the device 16 can be moved to a new location for taking an additional sample by repeating the steps above. Thus, multiple samples can be taken without removing the device from the body.

Referring to Fig. 2e, after a sufficient number of samples have been taken, and the device has been removed from the body, samples 1-5, stored in the space 25 in the order in which they were taken, can be recovered by again extending the inner tubular member 20 in the distal direction to force open the cutting elements 26, 26a. The stop member 23 is then extended distally (arrow 38) to push the samples sequentially from the end of the tubular member.

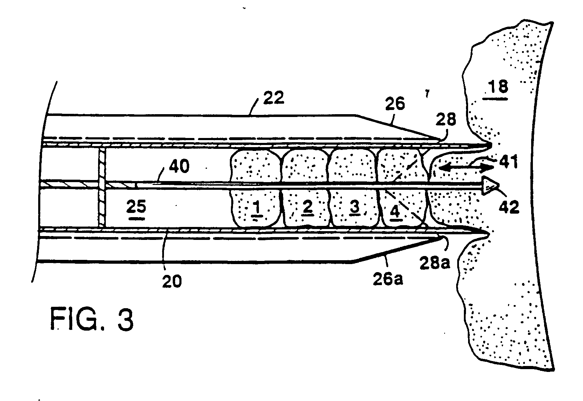

Referring to Fig. 3, another embodiment is shown. In this case, a retractor 40 is provided. The retractor member is axially movable (arrow 41), is formed of an extended length, and has a barbed tip 42 for piercing and retaining samples during axial travel. In use, the inner tubular member 20 is extended to open cutting elements 26, 26a and to rest against the tissue surface 18. The distal end of the tubular member may include, but does not require, a sharp cutting edge. The retractor 40 is then extended distally into the surface 18, thus displacing previous samples 1-4 proximally along its body. The retractor 40 is then withdrawn proximally drawing a piece of tissue into the distal end of the tubular member 20, as shown in Fig. 3. The cutting member 22 can then be moved distally to close the cutting elements 26, 26a and sever the sample from the surface 18. Other retractors can be used, such as hooks, tongues, and helical screw elements as described, for example, in "Multiple Biopsy Sampling Device", by Bruce H. Diamond, Donald E. Robinson, Alyssa J. Dassa, and Charles Warich, U.S. Serial No. 08/124,272, filed September 20, 1993 and "Multiple Biopsy Sampling Forceps" by Alyssa J. Dassa and Bruce H. Diamond, U.S. Serial No. 08/128,653, filed September 30, 1993.

The embodiment of Fig. 3 can also be used by providing the tubular member with a sharpened distal end and extending the tubular member 20 into the tissue, as described above with respect to Figs. 2 et seq. The retractor is positioned 50 it pierces the tissue that passes through the distal opening. However, rather than pushing the cutting member 22 in the distal direction to close the jaws, the tubular member 20 and retractor 40 are withdrawn proximally together relative to the moveable cutting elements, which close to sever the sample. The cutting member may be rotated as the inner tubular member is drawn proximally, to cause a shearing action that enhances cutting.

The features discussed above with respect to the various embodiments, e.g. arrangements for receiving tissue through the opening of the devices and arrangements for severing tissue from a surface, can be combined in further embodiments.

## Claims

1. A device (10) for collecting a sample of tissue (1-5) from a tissue surface (18), comprising:
a device body (20) having a distal end defining a forward-facing tissue receiving opening (19) through which tissue (1-5) passes when said opening (19) is near said tissue surface (18), and
a storage space (25) proximal of and adjacent said tissue receiving opening (19) for storage of multiple tissue samples (1-5); **characterised in that**
a set of movable, jaw-like cutting members (26, 26a) is disposed over said device body (20), actuatable to be opened and closed across said tissue receiving opening (19) when tissue from said surface (18) extends through said tissue receiving opening (19) for severing said tissue from said surface (18), whereby by repeatedly passing tissue (1-5) through said tissue receiving opening (19) and actuating said cutting members (26, 26a), multiple tissue samples can be stored within said storage space (25).

2. The device (10) of claim 1, wherein:
said cutting members (26, 26a) being coaxially disposed and axially positionable with respect to said tissue receiving opening (19) such that said device (10) is positionable between a first configuration in which the cutting members (26, 26a) are proximal of said opening (19) for receiving tissue (1-5) through said opening (19) and a second configuration in which the cutting members (26, 26a) are near said opening (19) where said cutting members (26, 26a) can be closed to sever tissue (1-5) and take said sample.

3. The device (10) of claim 1 or 2, wherein:
said cutting members (26, 26a) are biased closed,
said cutting members (26, 26a) are opened by disposing said device (10) in the first configuration, where said cutting members (26, 26a) bear on outer surfaces of the body (20) which oppose the force of said bias, and
said cutting members (26, 26a) are closed by disposing said device (10) in the second configuration where said cutting members (26, 26a) are free from said body (20) and close in response to said bias force.

4. The device (10) of claim 2 or 3, wherein said tissue receiving opening (19) is defined about its periphery by a tissue-cutting edge (24), said tissue passing through said opening (19) by urging said edge (24) distally into said tissue surface (18).

5. The device (10) of claim 1, 2, 3, or 4, further comprising an axially moveable retractor (40) for engaging said tissue (1-5) and drawing it proximally.

6. The device (10) of claim 5, wherein said retractor (40) is extendable distally beyond said opening (19) for engaging said tissue surface (18) and retractable proximally to draw tissue (1-5) through said tissue receiving opening (19).

7. The device (10) of claim 5 or 6, wherein said retractor (40) is a spear-form element adapted to pierce tissue (1-5) and has a retaining barb (42) on its distal end.

## Patentansprüche

1. Vorrichtung (10) zum Sammeln eine Gewebeprobe (1-5) von einer Gewebeoberfläche (18) aufweisend:
einen Vorrichtungskörper (20) mit einem distalen Ende, welches eine nach vorne gerichtete Gewebeaufnahmeöffnung (19) definiert, durch welche das Gewebe (1-5) passiert, wenn die Öffnung (19) sich in der Nähe der Gewebeoberfläche (18) befindet, und
einen Aufbewahrungsraum (25), der proximal zu der und in der Nähe zu der Gewebeaufnahmeüffnung (19) ist, für eine Aufbewahrung von mehreren Gewebeproben (1-5), **dadurch gekennzeichnet, dass** eine Gruppe von beweglichen, gebissartigen Schneideelementen (26, 26a) über dem Vorrichtungskörper (20) angeordnet ist, die betätigbar sind, um über die Gewebeaufnahmeöffnung (19) geöffnet und geschlossen zu werden, wenn sich Gewebe von der Oberfläche (18) durch die Gewebeaufnahmeöffnung (19) erstreckt, um das Gewebe von der Oberfläche (18) abzutrennen, wobei durch wiederholtes Durchtreten von Gewebe (1-5) durch die Gewebeaufnahmeöffnung (19) und Betätigen der Schneideelemente (26, 26a) mehrere Gewebeproben im Inneren des Aufbewahrungsraums (25) gespeichert werden können.

2. Vorrichtung (10) nach Anspruch 1, bei welcher die Schneideelemente (26, 26a) koaxial angeordnet und gegenüber der Gewebeaufnahmeöffnung (19) so axial positionierbar sind, dass die Vorrichtung (10) zwischen einer ersten Konfiguration, in welcher die Schneideelemente (26, 26a) sich proximal zu der Öffnung (19) zur Aufnahme von Gewebe (1-5) durch die Öffnung (19) befinden, und einer zweiten Konfiguration, in welcher sich die Schneideelemente (26, 26a) in der Nähe der Öffnung (19) befinden, in welcher die Schneideelemente (26, 26a) geschlossen werden können, um Gewebe (1-5) abzutrennen und die Probe zu entnehmen, positionierbar ist.

3. Vorrichtung (10) nach Anspruch 1 oder 2, bei welcher die Schneideelemente (26, 26a) unter Vorspannung geschlossen sind, die Schneideelemente (26, 26a) geöffnet werden, indem die Vorrichtung (10) in der ersten Konfiguration, in welcher die Schneideelemente (26, 26a) auf äußeren Oberflächen des Körpers (20) lagern, welche der Vorspannungskraft entgegen wirken, angeordnet ist, und die Schneideelemente (26, 26a) geschlossen werden, indem die Vorrichtung (10) in der zweiten Konfiguration, in welcher die Schneideelemente (26, 26a) frei von dem Körper (20) und genau in Antwort auf die Vorspannungskraft sind, angeordnet ist.

4. Vorrichtung (10) nach Anspruch 2 oder 3, bei welcher die Gewebeaufnahmeöffnung (19) um ihre Peripherie durch eine Gewebeschneidekante (24) definiert ist, wobei das Gewebe, durch die Öffnung (19) passiert, indem die Kante (24) distal in die Gewebeoberfläche (18) gezwungen wird.

5. Vorrichtung (10) nach Anspruch 1, 2, 3 oder 4, weiter aufweisend einen axial beweglichen Retraktor (40) für einen Eingriff mit dem Gewebe (1-5) und für ein proximales Herausziehen von diesem.

6. Vorrichtung (10) nach Anspruch 5, bei welcher der Retraktor (40) distal über die Öffnung (19) hinaus für einen Eingriff der Gewebeoberfläche (18) erstreckbar und proximal zurückziehbar ist, um Gewebe (1-5) durch die Gewebeaufnahmeöffnung (19) zu ziehen.

7. Vorrichtung (10) nach Anspruch 5 oder 6, bei welcher der Retraktor (40) ein speerartiges Element ist, welches dazu dient, Gewebe (1-5) zu durchstechen, und welches auf seinem distalen Ende einen Haltewiderhaken (42) aufweist.

## Revendications

1. Dispositif (10) pour recueillir un échantillon de tissu cellulaire (1-5) prélevé sur une surface tissulaire (18), comprenant :
un corps de dispositif (20) ayant une extrémité distale délimitant une ouverture réceptrice de tissu (19) dirigée vers l'avant, à travers laquelle le tissu (1-5) passe quand ladite ouverture (19) est à proximité de ladite surface tissulaire (18), et
un espace de stockage (25) proximal et adjacent par rapport à ladite ouverture réceptrice de tissu (19) pour le stockage de multiples échantillons de tissu cellulaire (1-5) ; **caractérisé en ce que**
un groupe d'organes tranchants (26, 26a), mobiles, semblables à des mâchoires, est disposé au-dessus dudit corps de dispositif (20), pouvant être actionnés pour s'ouvrir et se refermer à travers ladite ouverture réceptrice de tissu (19), quand du tissu de ladite surface (18) est étiré à travers ladite ouverture réceptrice de tissu (19) pour sectionner ledit tissu de ladite surface (18), de sorte que, le tissu (1-5) traversant à plusieurs reprises ladite ouverture réceptrice de tissu (19) et en actionnant lesdits organes tranchants (26, 26a), des échantillons multiples de tissu peuvent être stockés dans ledit espace de stockage (25).

2. Dispositif (10) selon la revendication 1, où :
lesdits organes tranchants (26, 26a), étant disposés de manière coaxiale et positionnables dans le sens axial par rapport à ladite ouverture réceptrice de tissu (19), de telle sorte que ledit dispositif (10) soit positionnable entre une première configuration, dans laquelle les organes tranchants (26, 26a) sont en position proximale par rapport à ladite ouverture (19) pour recueillir le tissu (1-5) à travers ladite ouverture (19), et une deuxième configuration, dans laquelle les organes tranchants (26,26a) sont à proximité de ladite ouverture (19), où lesdits organes tranchants (26, 26a) peuvent être refermés pour sectionner le tissu (1-5) et pour récupérer ledit échantillon.

3. Dispositif (10) selon la revendication 1 ou 2, dans lequel :
lesdits organes tranchants (26, 26a) sont rappelés en position de fermeture,
et lesdits organes tranchants (26, 26a) sont ouverts en disposant ledit dispositif (10) dans la première configuration, dans laquelle lesdits organes tranchants (26, 26a) portent contre des surfaces externes du corps du dispositif (20), lesquelles s'opposent à la force de rappel, et
lesdits organes tranchants (26, 26a) sont fermés en disposant ledit dispositif (10) dans la deuxième configuration, dans laquelle lesdits organes tranchants (26, 26a) sont dégagés du dit corps de dispositif (20) et se referment en réponse à ladite force du rappel.

4. Dispositif (10) selon la revendication 2 ou 3, dans lequel ladite ouverture réceptrice de tissu (19) est délimitée au niveau de sa périphérie par un bord tranchant pour couper le tissu (24), ledit tissu traversant ladite ouverture (19) alors que ledit bord (24) se trouve propulsé dans le sens distal contre ladite surface tissulaire (18).

5. Dispositif (10) selon la revendication 1, 2, 3 ou 4, comprenant en outre un écarteur (40) mobile dans le sens axial pour engager ledit tissu (1-5) et le tirer dans le sens proximal

6. Dispositif (10) selon la revendication 5, dans lequel ledit écarteur (40) est extensible dans le sens distal au-delà de ladite ouverture (19), pour faire s'engager ladite surface tissulaire (18), et rétractable dans le sens proximal pour tirer le tissu (1-5) à travers ladite ouverture réceptrice de tissu (19).

7. Dispositif (10) selon la revendication 5 ou 6, dans lequel ledit écarteur (40) est un élément, ayant la forme d'une aiguille, adapté pour percer le tissu (1-5) et est muni d'un ardillon de retenue (42) à son extrémité distale.
